# EUROPEAN PATENT APPLICATION

(11) **EP 4 394 555 A1**
(43) Date of publication of application: **03.07.2024**
(21) Application number: 22873046.1
(22) Date of filing: 27.09.2022
(51) Int. Cl.: G06F 3/01, A61B 5/055, A61B 10/00, A61M 21/00, G06Q 10/04

(54) **SENSATION TRANSMISSION SYSTEM, SENSATION TRANSMISSION METHOD, AND SENSATION TRANSMISSION PROGRAM**

(30) Priority: 27.09.2021 JP 2021156506; 27.09.2021 JP 2021156717; 27.09.2021 JP 2021157247
(71) Applicant: JVCKenwood Corporation, Yokohama-shi, Kanagawa 2210022 (JP)
(72) Inventor: SUGAHARA, Takayuki, Yokohama-shi, Kanagawa 221-0022 (JP); SHIMAKURA, Takamitsu, Yokohama-shi, Kanagawa 221-0022 (JP); ODAN, Shohei, Yokohama-shi, Kanagawa 221-0022 (JP)
(74) Representative: Wimmer, Hubert
(86) International application number: PCT/JP2022/035933
(87) International publication number: WO 2023/048295

(57) **Abstract**

A sensory transmission system includes a first apparatus configured to detect cerebral activation information on a first subject when the first subject performs perception, an estimation apparatus configured to estimate reference sensory information that is sensory information that is evoked in response to the perception based on the detected cerebral activation information on the first subject and estimate corresponding sensory information that is sensory information corresponding to the reference sensory information on a second subject different from the first subject based on the estimated reference sensory information, and a second apparatus configured to apply a stimulus to the second subject so as to evoke the estimated corresponding sensory information.

## Description

### Field

The present disclosure relates to a sensory transmission system, a sensory transmission method, and a sensory transmission program.

### Background

In recent years, a technology, such as functional magnetic resonance or near-infrared spectroscopy, for measuring cerebral activation information in a non-invasive manner has been developed, and a technology for a brain-machine interface that is an interface between a brain and an external apparatus is becoming realistic. As an example using the technology as described above, a certain configuration is disclosed that detects cerebral activation information on a sleeping subject, identifies a sleeping state of the subject, and applies a dream-induction stimulus to the subject when it is identified that the subject is in the REM sleep state (for example, see Patent Literature 1).

### Citation List

### Patent Literature

Japanese Laid-open Patent Publication No. 2003-332251 A

### Summary

### Technical Problem

A correspondence relationship between a brain activity state of a subject and measured cerebral activation information varies depending on a personality of the subject, a surrounding environment of the subject at the time of measurement, or the like. For example, in the technology described in Patent Literature 1, a type of dream that the subject has when the dream induction stimulus is applied varies depending on each subject, a timing at which the stimulus is applied, or the like, and it is difficult to control details of the dream. In contrast, when a brain activity state is identified or controlled based on cerebral activation information on a subject, there is a need for a technology that takes into account a certain factor, such as individual differences or differences in surrounding environments of subjects.

The present disclosure has been conceived in view of the foregoing situation, and an object of the present disclosure is to provide a sensory transmission system and a sensory transmission method capable of appropriately transmitting a sense between subjects when there are individual differences among the subjects or when there are differences in surrounding environments of the subjects. Solution to Problem

A sensory transmission system according to the present disclosure includes: a first apparatus configured to detect cerebral activation information on a first subject when the first subject performs perception; an estimation apparatus configured to estimate reference sensory information that is sensory information that is evoked in response to the perception based on the detected cerebral activation information on the first subject and estimate corresponding sensory information that is sensory information corresponding to the reference sensory information on a second subject different from the first subject based on the estimated reference sensory information; and a second apparatus configured to apply a stimulus to the second subject so as to evoke the estimated corresponding sensory information.

A sensory transmission method according to the present disclosure includes: detecting cerebral activation information on a first subject when the first subject performs perception; estimating reference sensory information that is sensory information that is evoked in response to the perception based on the detected cerebral activation information on the first subject; estimating corresponding sensory information that is sensory information corresponding to the reference sensory information on a second subject different from the first subject based on the estimated reference sensory information; and applying a stimulus to the second subject so as to evoke the estimated corresponding sensory information.

A sensory transmission program according to the present disclosure causes a computer to perform processing including: detecting cerebral activation information on a first subject when the first subject performs perception; estimating reference sensory information that is sensory information that is evoked in response to the perception based on the detected cerebral activation information on the first subject; estimating corresponding sensory information that is sensory information corresponding to the reference sensory information on a second subject different from the first subject based on the estimated reference sensory information; and applying a stimulus to the second subject so as to evoke the estimated corresponding sensory information.

### Advantageous Effects of Invention

According to the present disclosure, it is possible to appropriately transmit a sense between subjects when there are individual differences among the subjects or when there are differences in surrounding environments of the subjects.

### Brief Description of Drawings

FIG. 1 is a schematic diagram illustrating an example of a sensory transmission system according to a first embodiment.
FIG. 2 is a functional block diagram illustrating an example of the sensory transmission system according to the first embodiment.
FIG. 3 is a diagram illustrating an example of a neural network.
FIG. 4 is a diagram schematically illustrating an example of operation performed by the sensory transmission system according to the first embodiment.
FIG. 5 is a flowchart illustrating an example of operation performed by the sensory transmission system according to the first embodiment.
FIG. 6 is a diagram schematically illustrating an example of operation performed by a sensory transmission system according to a second embodiment.
FIG. 7 is a diagram schematically illustrating an example of operation performed by a sensory transmission system according to a third embodiment.
FIG. 8 is a functional block diagram illustrating an example of a sensory transmission system according to a fourth embodiment.
FIG. 9 is a diagram schematically illustrating an example of a sensory transmission system according to a fourth embodiment.
FIG. 10 is a flowchart illustrating an example of operation of the sensory transmission system according to the fourth embodiment.
FIG. 11 is a functional block diagram illustrating an example of a sensory transmission system according to a fifth embodiment.
FIG. 12 is a diagram schematically illustrating an example of operation performed by the sensory transmission system according to the fifth embodiment.
FIG. 13 is a flowchart illustrating an example of the operation performed by the sensory transmission system according to the fifth embodiment.

### Description of Embodiments

Embodiments of the present disclosure will be described below based on the drawings. The present invention is not limited by the embodiments below. In addition, components in the embodiments described below include one that can easily be thought of or replaced by a person skilled in the art and one that is practically identical.

### First Embodiment

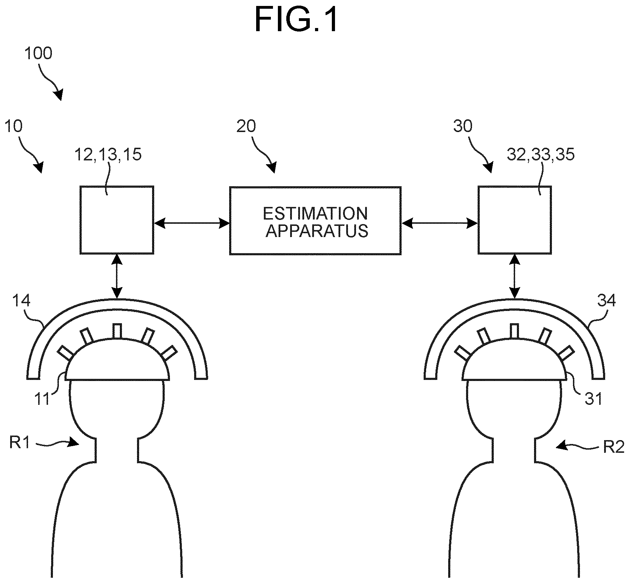

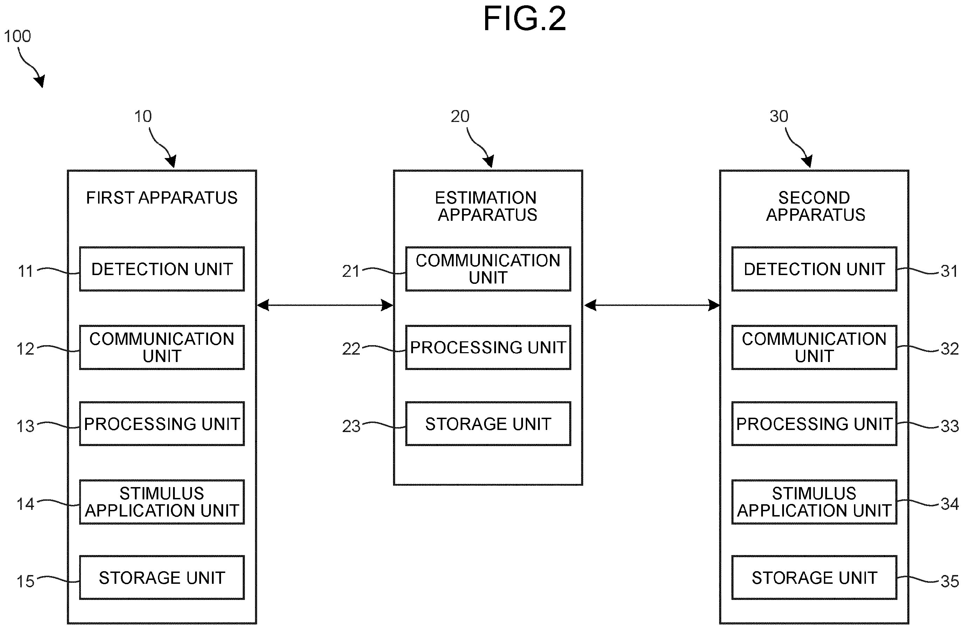

FIG. 1 is a schematic diagram illustrating an example of a sensory transmission system 100 according to a first embodiment. FIG. 2 is a functional block diagram illustrating an example of the sensory transmission system 100. As illustrated in FIG. 1 and FIG. 2, the sensory transmission system 100 includes a first apparatus 10, an estimation apparatus 20, and a second apparatus 30.

The first apparatus 10 detects cerebral activation information on a first subject R1 when a first subject R1 performs perception. The first apparatus 10 includes a detection unit 11, a communication unit 12, a processing unit 13, a stimulus application unit 14, and a storage unit 15.

The detection unit 11 detects the cerebral activation information. Examples of the cerebral activation information include concentration of oxygenated hemoglobin, concentration of deoxidized hemoglobin, and concentration of total hemoglobin that are included in a cerebral blood flow of the subject. As the detection unit 11, for example, it may be possible to use a measurement apparatus that performs measurement based on the principle of functional Magnetic Resonance Imaging (fMRI), functional Near-Infrared Spectroscopy (fNIRS), or the like, a measurement apparatus that uses an invasive electrode, a measurement apparatus for which a micromachine is arranged in a cerebral blood vessel and which performs measurement by the micromachine, or the like. Meanwhile, the detection unit 11 is not limited to the apparatus as described above, but it may be possible to use a different kind of apparatus. The cerebral activation information may be represented by, for example, a magnitude of an activity level for each voxel when a brain of the first subject R1 is segmented by a three-dimensional matrix that is composed of voxels of few millimeters or less.

The communication unit 12 is an interface that performs wired communication or wireless communication. The communication unit 12 transmits the brain activation information that is detected by the detection unit 11 to the estimation apparatus 20. The communication unit 12 includes an interface for performing communication with an external apparatus by what is called a wireless local area network (LAN) that is compliant with IEEE 802.11 standards. The communication unit 12 may implement communication with the external apparatus by being controlled by a processing unit 22. Meanwhile, the communication method is not limited to the wireless LAN, but may include, for example, a wireless communication method, such as an infrared communication method, a Bluetooth (registered trademark) communication method, or a Wireless USB. Furthermore, it may be possible to adopt a wired connection, such as a USB cable, HDMI (registered trademark), IEEE1394, or Ethernet.

The processing unit 13, the stimulus application unit 14, and the storage unit 15 will be described later. In the first embodiment, the processing unit 13, the stimulus application unit 14, and the storage unit 15 need not always be arranged.

The estimation apparatus 20 includes a communication unit 21, the processing unit 22, and a storage unit 23. The communication unit 21 is an interface that performs wired communication or wireless communication. The communication unit 21 receives, for example, the cerebral activation information that is transmitted from the first apparatus 10. The communication unit 21 transmits, for example, corresponding sensory information that is estimated by the processing unit 22 (to be described later) to the second apparatus 30. Meanwhile, the communication unit 21 may have the same configuration as the communication unit 12 as described above.

The processing unit 22 includes a processing apparatus, such as a Central Processing Unit (CPU), and a storage apparatus, such as a Random Access Memory (RAM) or a Read Only Memory (ROM). The processing unit 22 performs various kinds of processes including an estimation process to be described below.

The storage unit 23 stores therein various kinds of information. The storage unit 23 includes, for example, a storage, such as a hard disk drive or a solid state drive. Meanwhile, it may be possible to use, as the storage unit 23, an external storage medium, such as a removable disk.

The processing unit 22 estimates sensory information (reference sensory information) on a sense that the first subject R1 has evoked in response to a perception, based on the detected cerebral activation information on the first subject R1. The sensory information may be, for example, information on at least one of five senses, such as a sense of sight, a sense of hearing, a sense of touch, a sense of taste, and a sense of smell, a sense of balance, a somatosensory system other than the above-described senses, or the like. Specifically, when the sensory information is information on the sense of sight, the sensory information is image data that is perceived by the first subject R1; however, the sensory information is not limited to this example, and may be image data that is obtained by performing sampling on original image data instead of the original image data or image data that is obtained by performing a filtering process on the original image data. Further, when the sensory information is information on the sense of sight, the sensory information may be information on light that enters an eyeball of the first subject R1. In this case, for example, it may be possible to acquire the information on light by a contact lens that includes an optical sensor. Alternatively, it may be possible to acquire the information on light by using an artificial retina. Specifically, it may be possible to use information on light of a charge coupled device (CCD) sensor that is arranged on the artificial retina. Furthermore, when the sensory information is information on the sense of hearing, the sensory information may be voice signal data that is perceived by the first subject R1. Moreover, when the sensory information is information on the sense of taste, the sensory information may be data that indicates indices of a plurality of chemical substances that reproduce the sense of taste that is perceived by the first subject R1. Furthermore, when the sensory information is information on the sense of touch, the sensory information may be data in which a whole body surface of the first subject R1 is developed on a plane and which indicates a portion and a degree of occurrence of a stimulus in the developed view. Meanwhile, the pieces of sensory information as described above are mere examples, and embodiments are not limited to these examples. With respect to the first subject R1, a correspondence relationship between a type of sensory information and a type of cerebral activation information that is observed when each type of sensory information is evoked may be obtained by performing an experiment in advance. For example, it is possible to generate a first trained model by associating the cerebral activation information that is detected from the first subject R1 and sensory information that corresponds to the cerebral activation information, adopting the associated pieces of information as a single training data set, and performing machine learning on the training data set. The first trained model may be stored in, for example, the storage unit 23.

Furthermore, the processing unit 22 estimates corresponding sensory information that is sensory information corresponding to reference sensory information on a second subject R2 that is different from the first subject R1 based on the estimated reference sensory information. Meanwhile, specific examples of the corresponding sensory information may be equivalent to specific examples of the sensory information. The second subject R2 is a subject who serves as a transmission destination to which a sense of the first subject R1 is transmitted. As a relationship between the reference sensory information and the corresponding sensory information, it is possible to associate the pieces of information by performing an experiment in advance. For example, it is possible to generate a second trained model by adopting, as a single training data set, pieces of sensory information that correspond to each other between the first subject R1 and the second subject R2 and performing machine learning on the training data set. The second trained model may be stored in, for example, the storage unit 23.

The second apparatus 30 applies a stimulus to the second subject R2 so as to evoke the estimated corresponding sensory information. The second apparatus 30 includes a detection unit 31, a communication unit 32, a processing unit 33, a stimulus application unit 34, and a storage unit 35.

The detection unit 31 will be described later. In the first embodiment, the detection unit 31 need not always be arranged.

The communication unit 32 is an interface that performs wired communication or wireless communication. The communication unit 32 receives the corresponding sensory information that is transmitted from the estimation apparatus 20. Further, the communication unit 32 transmits the cerebral activation information that is detected by the detection unit 31 to the estimation apparatus 20. Meanwhile, the communication unit 32 may have the same configuration as the communication unit 12 as described above.

The stimulus application unit 34 applies a stimulus to the second subject R2 by applying an electromagnetic wave signal to a target part in a brain of the second subject R2 and activating the target part. In this case, the brain of the second subject R2 is segmented by a three-dimensional matrix that is composed of voxels of few millimeters or less and an electromagnetic wave is applied for each of the voxels, for example. The stimulus application unit 34 is able to apply the electromagnetic wave based on stimulus image information that indicates a voxel in the three-dimensional matrix to which the electromagnetic wave is to be applied and intensity of the electromagnetic wave to be applied. A size, a position, and the like of the voxel in the three-dimensional matrix of the stimulus image information may correspond to those of a voxel of a three-dimensional matrix of the cerebral activation information, for example. It is possible to obtain a correspondence relationship among a voxel in the brain of the second subject R2 to which the electromagnetic wave is to be applied, intensity of the electromagnetic wave, and a type of sensory information to be evoked, by performing an experiment in advance. For example, it is possible to generate a third trained model by associating the stimulus image information on the second subject R2 and sensory information that the second subject R2 evokes when the electromagnetic wave is applied based on the stimulus image information, adopting the pieces of associated information as a single training data set, and performing machine learning on the training data set. The third trained model may be stored in, for example, the storage unit 35 of the second apparatus 30. The processing unit 33 is able to calculate the stimulus image information that corresponds to the received corresponding sensory information based on the corresponding sensory information that is received by the communication unit 32 and the third trained model.

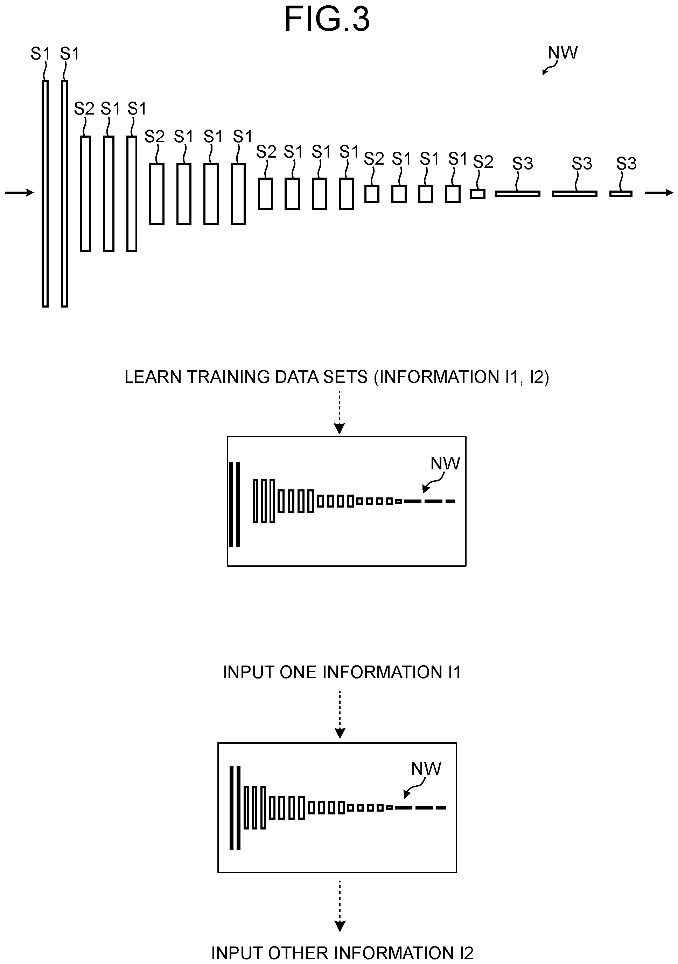

The first trained model, the second trained model, and the third trained model as described above may be generated by using, for example, a neural network (convolutional neural network) that is represented by VGG16. FIG. 3 is a diagram illustrating an example of the neural network. As illustrated in an upper part in FIG. 3, a neural network NW includes 13 convolutional layers S1, five pooling layers S2, and three fully-connected layers S3. The neural network performs processes on input information in the convolutional layers S1 and the pooling layers S2 in sequence, and processing results are connected by the fully-connected layers S3 and then output.

When the first trained model, the second trained model, and the third trained model are to be generated, as illustrated in a middle part in FIG. 3, training data sets including pieces of information (I1 and I2) that correspond to each other are input to the neural network NW, and a correlation between the training data sets is learned by machine learning, such as deep learning. In other words, the neural network NW is optimized by training and the trained models are generated. For example, training is performed such that one of the pieces of information included in the respective training data sets is solved when the other one of the pieces of information is input.

When estimation is to be performed by using the first trained model, the second trained model, and the third trained model, as illustrated in the lower part in FIG. 3, the information I1 that is one of the two pieces of information included in the respective training data sets is input to the neural network NW. The first trained model, the second trained model, and the third trained model output the information I2 that is the other one of the pieces of information and that corresponds to the input information I1 that is one of the pieces of information, based on a learning result of the correlation between the pieces of information included in the training data sets. Meanwhile, in the present embodiment, the example is described in which the trained models are generated by using the convolutional neural network that is represented by VGG16, but embodiments are not limited to this example, and it may be possible to generate the trained models by using a different kind of neural network.

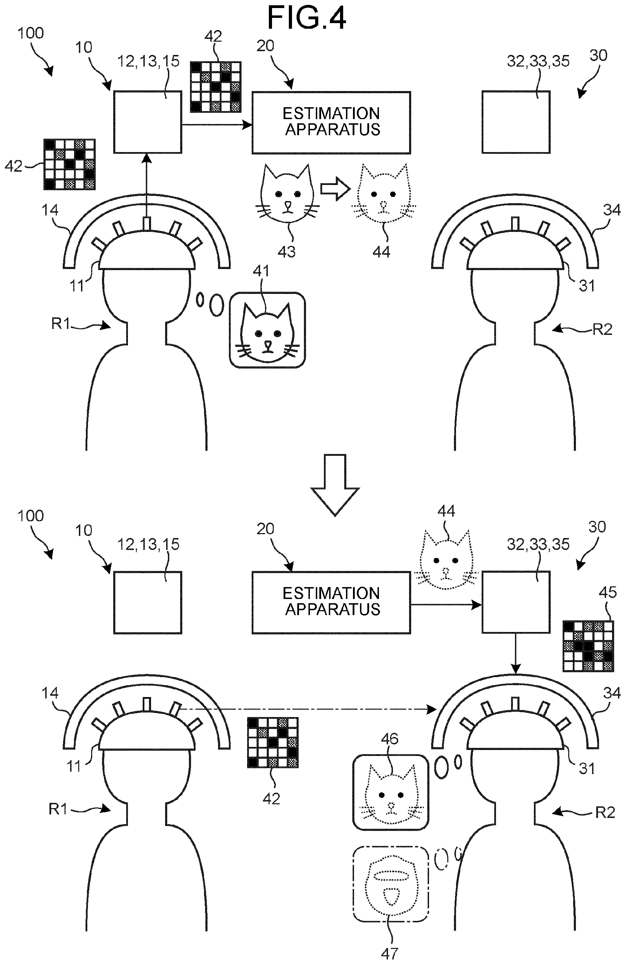

A sensory transmission method using the sensory transmission system 100 that is configured as described above will be described below. FIG. 4 is a diagram schematically illustrating an example of operation performed by the sensory transmission system 100. As illustrated in FIG. 4, the first subject R1 is caused to perform perception so as to evoke the reference sensory information. In the following, an example will be described in which the first subject R1 visually perceives a cat face and evokes visual information.

As illustrated in an upper pert in FIG. 4, the detection unit 11 detects cerebral activation information 42 on the first subject R1 who perceives a cat face and evokes sensory information 41. The communication unit 12 transmits the cerebral activation information 42 that is detected by the detection unit 11 to the estimation apparatus 20.

In the estimation apparatus 20, the communication unit 21 receives the cerebral activation information 42 that is transmitted from the first apparatus 10. The processing unit 22 estimates reference sensory information 43 based on the received cerebral activation information 42. In this case, the processing unit 22 inputs the cerebral activation information 42 on the first subject R1 to the first trained model. The first trained model outputs the reference sensory information 43 that corresponds to the input cerebral activation information 42 based on a learning result of a correlation between the cerebral activation information 42 and the reference sensory information 43. The processing unit 22 acquires the output reference sensory information 43 as an estimation result.

The processing unit 22 estimates corresponding sensory information 44 that corresponds to the reference sensory information 43 with respect to the second subject R2 based on the estimated reference sensory information 43. In this case, the processing unit 22 inputs the acquired reference sensory information 43 to the second trained model. The second trained model outputs the corresponding sensory information 44 that corresponds to the input reference sensory information 43 based on a learning result of a correlation between the reference sensory information 43 and the corresponding sensory information 44. The processing unit 22 acquires the output corresponding sensory information 44 as an estimation result. The communication unit 21 transmits the acquired corresponding sensory information 44 to the second apparatus 30.

In the second apparatus 30, the communication unit 32 receives the corresponding sensory information 44 that is transmitted from the estimation apparatus 20. The processing unit 33 inputs the received corresponding sensory information 44 to the third trained model that is stored in the storage unit 35. The third trained model outputs stimulus image information 45 that corresponds to the input corresponding sensory information 44 based on a learning result of a correlation between the corresponding sensory information 44 and the stimulus image information. The stimulus application unit 34 applies a stimulus to the second subject R2 by applying an electromagnetic wave to the brain of the second subject R2 based on the output stimulus image information 45. As a result, the second subject R2 to whom the stimulus is applied by the stimulus application unit 34 evokes corresponding sensory information 46 that corresponds to the stimulus image information 45. In other words, the second subject R2 evokes visual information on the cat face as the corresponding sensory information 46.

Meanwhile, the first subject R1 and the second subject R2 have different personalities or the like, and therefore, correspondence relationships between brain activity states and the cerebral activation information vary. Therefore, as illustrated in a lower part in FIG. 4 (indicated by a chain line), if the cerebral activation information 42 on the first subject R1 is directly transmitted to the second apparatus 30 and the second apparatus 30 applies a stimulus to the brain of the second subject R2 so as to correspond to the cerebral activation information 42, the second subject R2 is more likely to evoke different visual information from the cat face as sensory information 47. In this case, the sensory information on the first subject R1 is not appropriately transmitted to the second subject R2.

In contrast, in the sensory transmission system 100 of the present embodiment, the estimation apparatus 20 estimates the corresponding sensory information 44 on the second subject R2, so that the visual information on the cat face is appropriately transmitted from the first subject R1 to the second subject R2.

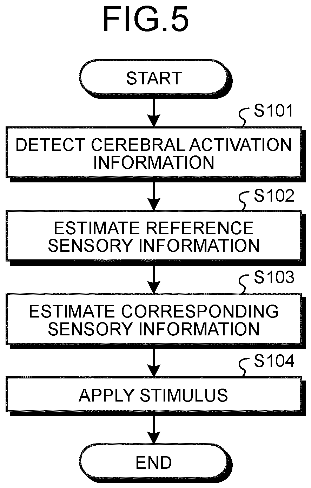

FIG. 5 is a flowchart illustrating operation performed by the sensory transmission system 100. As illustrated in FIG. 5, in the sensory transmission system 100, the first apparatus 10 detects the cerebral activation information on the first subject R1 when the first subject R1 performs perception (Step S101). Subsequently, the estimation apparatus 20 estimates the reference sensory information that the first subject R1 has evoked in response to the perception, based on the cerebral activation information on the first subject R1 (Step S102). Then, the estimation apparatus 20 estimates the corresponding sensory information that corresponds to the reference sensory information on the second subject R2 that is different from the first subject R1, based on the estimated reference sensory information (Step S103). Further, the second apparatus 30 applies a stimulus to the second subject R2 so as to evoke the estimated corresponding sensory information (Step S104).

As described above, the sensory transmission system 100 according to the present embodiment includes the first apparatus 10 that detects cerebral activation information on the first subject R1 when the first subject R1 performs perception, the estimation apparatus 20 that estimates reference sensory information that is sensory information that is evoked in response to the perception based on the detected cerebral activation information on the first subject R1 and estimates corresponding sensory information that is sensory information corresponding to the reference sensory information on the second subject R2 different from the first subject R1 based on the estimated reference sensory information, and the second apparatus 30 that applies a stimulus to the second subject R2 so as to evoke the estimated corresponding sensory information.

The sensory transmission method according to the present embodiment includes detecting cerebral activation information on the first subject R1 when the first subject R1 performs perception, estimating reference sensory information that the first subject R1 has evoked in response to the perception based on the detected cerebral activation information on the first subject R1 and estimating corresponding sensory information that corresponds to the reference sensory information on the second subject R2 different from the first subject R1 based on the estimated reference sensory information, and applying a stimulus to the second subject R2 so as to evoke the estimated corresponding sensory information.

A sensory transmission program according to the present embodiment causes a computer to perform processing including: detecting cerebral activation information on the first subject R1 when the first subject R1 performs perception; estimating reference sensory information that the first subject R1 has evoked in response to the perception based on the detected cerebral activation information on the first subject R1; estimating corresponding sensory information that corresponds to the reference sensory information on the second subject R2 different from the first subject R1 based on the estimated reference sensory information; and applying a stimulus to the second subject R2 so as to evoke the estimated corresponding sensory information.

With this configuration, the second subject R2 is not caused to evoke the reference sensory information on the first subject R1 as it is, but the corresponding sensory information that corresponds to the second subject R2 is estimated based on the reference sensory information and a stimulus is applied to the second subject R2 so as to evoke the estimated corresponding sensory information; therefore, even when brain activities are different between the first subject R1 and the second subject R2 when the sensory information is evoked, it is possible to appropriately transmit the sensory information from the first subject R1 to the second subject R2.

In the sensory transmission system 100 according to the present embodiment, the reference sensory information is sensory information that is evoked by the first subject R1 for whom the cerebral activation information is detected. With this configuration, the sensory information on the first subject R1 and the sensory information on the second subject R2 are directly associated with each other, so that it is possible to appropriately transmit the sensory information.

In the sensory transmission system 100 according to the present embodiment, the stimulus includes activation of a target part by application of an electromagnetic wave signal to the target part in the brain of the second subject R2. With this configuration, by directly activating the brain of the second subject R2, it is possible to cause the second subject R2 to more directly evoke the sensory information.

### Second Embodiment

A second embodiment will be described below. In the first embodiment, the example has been described in which the sensory transmission system 100 transmits a sense in one direction from the first subject R1 to the second subject R2. In contrast, in the second embodiment, the sensory transmission system 100 transmits a sense even from the second subject R2 to the first subject R1. In other words, the sensory transmission system 100 is configured so as to be able to bi-directionally transmit a sense between the first subject R1 and the second subject R2.

An entire configuration of the sensory transmission system 100 is the same as the first embodiment. In the following, the configuration of the sensory transmission system 100 will be described from the viewpoint of the second apparatus 30 with reference to FIG. 1 and FIG. 2.

The second apparatus 30 includes the detection unit 31, the communication unit 32, the processing unit 33, the stimulus application unit 34, and the storage unit 35. The processing unit 33, the stimulus application unit 34, and the storage unit 35 are the same as those of the first embodiment. The detection unit 31 detects the cerebral activation information on the second subject R2 similarly to the detection unit 11 of the first embodiment. The communication unit 32 transmits the cerebral activation information that is detected by the detection unit 31 to the estimation apparatus 20.

The estimation apparatus 20 includes, similarly to the estimation apparatus 20 of the first embodiment, the communication unit 21, the processing unit 22, and the storage unit 23. The communication unit 21 is able to perform wired communication or wireless communication. In the present embodiment, the communication unit 21 receives, for example, the cerebral activation information that is transmitted from the second apparatus 30. The communication unit 21 transmits, for example, corresponding sensory information that is estimated by the processing unit 22 (to be described later) to the first apparatus 10.

The processing unit 22 estimates sensory information (reference sensory information) on a sense that the second subject R2 has evoked in response to a perception, based on the detected cerebral activation information on the second subject R2. With respect to the second subject R2, a correspondence relationship between a type of sensory information and a type of cerebral activation information that is observed when each type of sensory information is evoked may be obtained by performing an experiment in advance. For example, it is possible to generate a fourth trained model by associating the cerebral activation information that is detected from the second subject R2 and sensory information that corresponds to the cerebral activation information, adopting the associated pieces of information as a single training data set, and performing machine learning on the training data set. The fourth trained model may be stored in, for example, the storage unit 23.

Furthermore, the processing unit 22 estimates corresponding sensory information that is sensory information corresponding to reference sensory information on the first subject R1 based on the estimated reference sensory information. In this case, the processing unit 22 is able to perform estimation based on the second trained model that is stored in the storage unit 23.

The first apparatus 10 includes the detection unit 11, the communication unit 12, the processing unit 13, the stimulus application unit 14, and the storage unit 15. The detection unit 11 and the communication unit 12 have the same configurations as those of the first embodiment. Meanwhile, the communication unit 12 receives the corresponding sensory information that is transmitted from the estimation apparatus 20.

The processing unit 13 estimates stimulus image information that corresponds to the corresponding sensory information that is received by the communication unit 12. The stimulus image information is information indicating a detail of a stimulus that is applied to the first subject R1 by the stimulus application unit 14.

The stimulus application unit 14 applies a stimulus to the first subject R1 by applying an electromagnetic wave signal to a target part in a brain of the first subject R1 and activating the target part. In this case, similarly to the stimulus application unit 34 of the first embodiment, the brain of the first subject R1 is segmented into a three-dimensional matrix that is composed of voxels of few millimeters or less and an electromagnetic wave is applied for each of the voxels, for example. The stimulus application unit 14 is able to apply electromagnetic wave based on stimulus image information that indicates a voxel in the three-dimensional matrix to which the electromagnetic wave is to be applied and intensity of the electromagnetic wave to be applied. It is possible to obtain a correspondence relationship among a voxel in the brain of the first subject R1 to which electromagnetic wave is to be applied, intensity of the electromagnetic wave, and a type of sensory information to be evoked, by performing an experiment in advance. For example, it is possible to generate a fifth trained model by associating the stimulus image information on the first subject R1 and sensory information that the first subject R1 evokes when the electromagnetic wave is applied based on the stimulus image information, adopting the pieces of associated information as a single training data set, and performing machine learning on the training data set. The fifth trained model may be stored in, for example, the storage unit 15 of the first apparatus 10.

The fourth trained model and the fifth trained model as described above may be generated by using, for example, a neural network that is represented by VGG16, similarly to the first trained model to the third trained model. When the fourth trained model and the fifth trained model are to be generated, respective training data sets are input to the neural network, and a correlation between the training data sets is learned by machine learning, such as deep learning. In other words, the neural network is optimized by training and the trained models are generated. Meanwhile, the neural network is not limited to the convolutional neural network that is represented by VGG16, but it may be possible to generate the trained models by using a different kind of neural network.

A sensory transmission method using the sensory transmission system 100 that is configured as described above will be described below. Meanwhile, the sensory transmission method for transmitting a sense from the first subject R1 to the second subject R2 is the same as the first embodiment. In the present embodiment, a case will be described in which a sense is transmitted from the second subject R2 to the first subject R1.

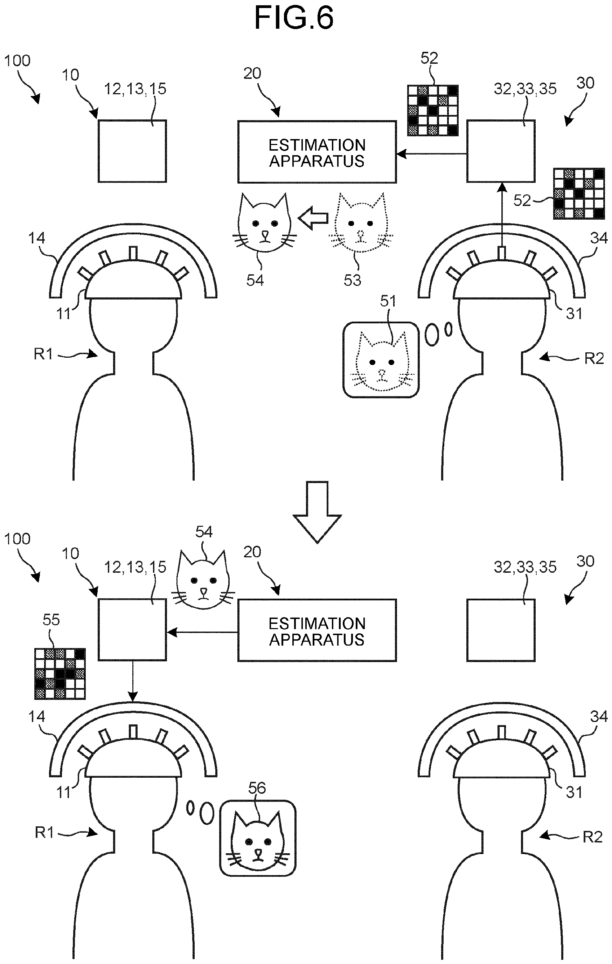

FIG. 6 is a diagram schematically illustrating an example of operation performed by the sensory transmission system 100. As illustrated in FIG. 6, the second subject R2 is caused to perform perception so as to evoke the reference sensory information. In the following, an example will be described in which the second subject R2 visually perceives a cat face and evokes visual information. Meanwhile, in the example below, the cat face is the same as the cat face of the first embodiment.

As illustrated in an upper part in FIG. 6, the detection unit 31 detects cerebral activation information 52 on the second subject R2 who perceives the cat face and evokes sensory information 51. The communication unit 32 transmits the cerebral activation information 52 that is detected by the detection unit 31 to the estimation apparatus 20.

In the estimation apparatus 20, the communication unit 21 receives the cerebral activation information 52 that is transmitted from a third apparatus 130. The processing unit 22 estimates reference sensory information 53 based on the received cerebral activation information 52. In this case, the processing unit 22 inputs the cerebral activation information 52 on the second subject R2 to the fourth trained model. The fourth trained model outputs the reference sensory information 53 that corresponds to the input cerebral activation information 52 based on a learning result of a correlation between the cerebral activation information 52 and the reference sensory information 53. The processing unit 22 acquires the output reference sensory information 53 as an estimation result.

The processing unit 22 estimates corresponding sensory information 54 that corresponds to the reference sensory information 53 with respect to the first subject R1 based on the estimated reference sensory information 53. In this case, the processing unit 22 inputs the acquired reference sensory information 53 to the second trained model. The second trained model outputs the corresponding sensory information 54 that corresponds to the input reference sensory information 53 based on a learning result of a correlation between the reference sensory information 53 and the corresponding sensory information 54. The processing unit 22 acquires the output corresponding sensory information 54 as an estimation result. The communication unit 21 transmits the acquired corresponding sensory information 54 to the first apparatus 10.

As illustrated in a lower part in FIG. 6, in the first apparatus 10, the communication unit 12 receives the corresponding sensory information 54 that is transmitted from the estimation apparatus 20. The processing unit 13 inputs the received corresponding sensory information 54 to the fifth trained model that is stored in the storage unit 15. The fifth trained model outputs stimulus image information 55 that corresponds to the input corresponding sensory information 54 based on a learning result of a correlation between the corresponding sensory information 54 and the stimulus image information 55. The stimulus application unit 14 applies a stimulus to the first subject R1 by applying an electromagnetic wave to the brain of the first subject R1 based on the output stimulus image information 55. As a result, the first subject R1 to whom the stimulus is applied by the stimulus application unit 14 evokes corresponding sensory information 56 that corresponds to the stimulus image information 55. In other words, the first subject R1 evokes the visual information on the cat face as the corresponding sensory information 56. In this manner, the visual information on the cat face is transmitted from the second subject R2 to the first subject R1.

As described above, in the sensory transmission system 100 according to the present embodiment, the reference sensory information is sensory information that is evoked by the second subject R2 for whom the cerebral activation information is detected. With this configuration, the sensory information on the second subject R2 and the sensory information on the first subject R1 are directly associated with each other, so that it is possible to appropriately transmit the sensory information between subjects who have different brain activities.

### Third Embodiment

A third embodiment will be described below. In the first embodiment and the second embodiment as described above, the sensory information that is evoked by the subject for whom the cerebral activation information is detected is adopted as the reference sensory information. In contrast, in the third embodiment, an example will be described in which standard sensory information that is extracted based on pieces of sensory information that correspond to one another among a plurality of subjects is adopted as the reference sensory information. An entire configuration of the sensory transmission system 100 is the same as the first embodiment.

In the estimation apparatus 20, when the corresponding sensory information is to be estimated from the reference sensory information, the standard sensory information is used as the reference sensory information. The standard sensory information may be, for example, an average value of pieces of cerebral activation information that are detected in a plurality of subjects when a plurality of detectors perform same perception, for example, when a plurality of detectors view the same image. Therefore, in the standard sensory information, individual differences due to acquired memories is reduced, and the standard sensory information serves as sensory information on an average person.

The standard sensory information may be extracted from a learning result when pieces of sensory information that correspond to one another other among the plurality of subjects is learned. For example, it is possible to generate a sixth trained mode by adopting, as a single training data set, the cerebral activation information on a specific subject (for example, the first subject R1 or the second subject R2) and the standard sensory information (an average value of pieces of cerebral activation information on a plurality of subjects) and performing machine learning on the training data sets. When the sixth trained model is to be generated, it may be possible to extract the standard sensory information from a plurality of pieces of sensory information included in the training data sets and associate the extracted standard sensory information and each piece of the cerebral activation information included in the training data sets. As a result, for example, the sixth trained model is generated such that the correspondence relationship between each piece of the brain activation information on the plurality of subjects and the standard sensory information is subjected to machine learning with respect to different kinds of sensory information, such as sensory information on the sense of sight when a certain target object is viewed, sensory information on the sense of hearing when certain sound is heard, or sensory information on the sense of touch when a certain target object is touched. The sixth trained model may be stored in, for example, a storage unit 25.

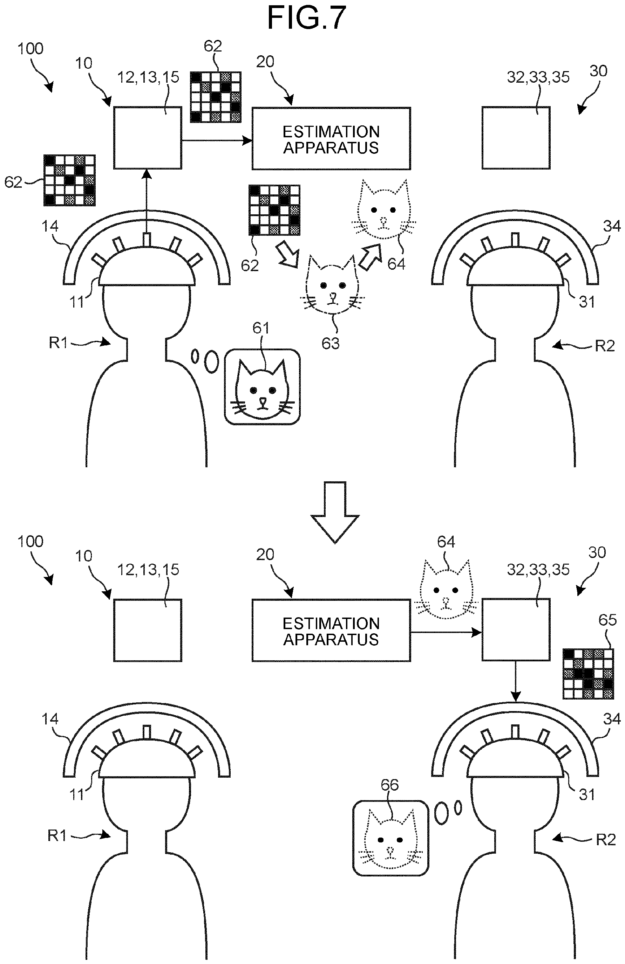

A sensory transmission method using the sensory transmission system 100 that is configured as described above will be described below. FIG. 7 is a diagram schematically illustrating an example of operation performed by the sensory transmission system 100. For example, when sensory information 61 that the first subject R1 has evoked in response to the perception is to be transmitted to the second subject R2, the first apparatus 10 acquires cerebral activation information 62 on the first subject R1 and transmits the cerebral activation information 62 to the estimation apparatus 20.

In the estimation apparatus 20, the processing unit 33 inputs the cerebral activation information 62 that is transmitted from the first apparatus 10 and identification information on the second subject R2 who is a transmission destination of the sensory information to the sixth trained model that is stored in the storage unit 35. The sixth trained model calculates standard sensory information 63 that corresponds to the cerebral activation information 62, and outputs, as corresponding sensory information 64, the sensory information on the second subject R2 associated with the standard sensory information 63. The communication unit 21 transmits the output corresponding sensory information 64 to the second apparatus 30.

The second apparatus 30 receives, similarly to the first embodiment, the corresponding sensory information 64 that is transmitted from the estimation apparatus 20, and acquires stimulus image information 65 based on the received corresponding sensory information 64. The stimulus application unit 34 applies a stimulus to the second subject R2 by applying an electromagnetic wave to the brain of the second subject R2 based on the output stimulus image information 65. The second subject R2 to whom the stimulus is applied by the stimulus application unit 34 evokes corresponding sensory information 66 that corresponds to the stimulus image information 65.

As described above, in the sensory transmission system 100 according to the present embodiment, the reference sensory information is the standard sensory information 63 that is extracted based on pieces of sensory information that correspond to one another among a plurality of subjects. With this configuration, the standard sensory information 63 that is extracted based on pieces of sensory information that correspond to one other among the plurality of subjects is used as the reference sensory information, so that it is possible to appropriately transmit the sensory information among a large number of subjects.

### Fourth Embodiment

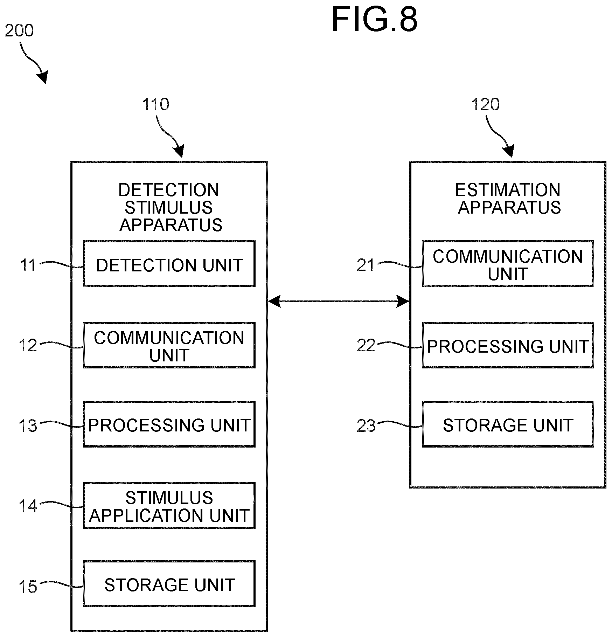

FIG. 8 is a diagram illustrating an example of a sensory transmission system 200 according to the fourth embodiment. In the sensory transmission system 100 of the embodiments as described above, the case has been described in which the sensory information is transmitted between different subjects. In contrast, in the sensory transmission system 200 described in the fourth embodiment, a case will be described in which the sensory information is transmitted in the same subject.

As illustrated in FIG. 8, the sensory transmission system 200 includes a detection stimulus apparatus (a detection apparatus or a stimulus apparatus) 110 and an estimation apparatus 120. The detection stimulus apparatus 110 has the same configuration as the first apparatus 10 described in the embodiments as described above, and includes, for example, the detection unit 11, the communication unit 12, the processing unit 13, the stimulus application unit 14, and the storage unit 15. The detection unit 11 detects cerebral activation information. The communication unit 12 performs wired communication or wireless communication, and transmits the cerebral activation information that is detected by the detection unit 11 to the estimation apparatus 20. The processing unit 13 calculates stimulus image information based on the corresponding sensory information that is received by the communication unit 12. The stimulus application unit 14 applies a stimulus to a target subject R4 by applying an electromagnetic wave signal to a target part in a brain of the target subject R4 based on the calculated stimulus image information and activating the target part. The storage unit 15 stores therein various kinds of information.

The estimation apparatus 120 includes the communication unit 21, the processing unit 22, and the storage unit 23. The communication unit 21 is able to perform wired communication or wireless communication. In the present embodiment, the communication unit 21 receives, for example, the cerebral activation information that is transmitted from the detection stimulus apparatus 110. The communication unit 21 transmits, for example, corresponding sensory information that is estimated by the processing unit 22 (to be described later) to the detection stimulus apparatus 110.

The processing unit 22 estimates the reference sensory information that is evoked in response to a perception, based on the cerebral activation information on the target subject R4 that is detected at a first time point. The first time point may be a time point at which the target subject R4 was a young child and may be a time point at which the target subject R4 was younger than 3 years old, for example.

As the reference sensory information, for example, it is possible to adopt the sensory information that the target subject R4 evoked in response to a perception at the first time point. In this case, the processing unit 22 is able to estimate the reference sensory information based on a seventh trained model that is the same as the first trained model as described above. The seventh trained model may be stored in, for example, the storage unit 25.

Furthermore, it may be possible to adopt, as the reference sensory information, the standard sensory information that is extracted based on sensory information that correspond to one another among a plurality of subjects, for example. As the standard sensory information, for example, it may be possible to adopt information that is extracted based on sensory information that a plurality of subjects who have brain growth conditions equivalent to the target subject R4. Examples of the plurality of subjects as described above include a plurality of subjects with equivalent ages, a plurality of subjects who have grown in equivalent environments (latitude, cultural environments, used languages, or the like), and a plurality of subjects with the same or equivalent occupations. The processing unit 22 is able to use an eighth trained model that estimates the standard sensory information based on the cerebral activation information on the target subject R4 at the first time point, for example. The eighth trained model may be stored in, for example, the storage unit 25.

Moreover, the processing unit 22 estimates the corresponding sensory information that corresponds to the reference sensory information on the target subject R4 at a second time point that is later than the first time point, based on the estimated reference sensory information. A degree of growth of the brain of the target subject R4 may largely vary between the first time point and the second time point, for example. For example, it may be possible to generate a ninth trained model by adopting, as a single training data set, pieces of sensory information that correspond to each other between the target subject R4 at the first time point and the target subject R4 at the second time point and performing machine learning on the training data set. The ninth trained model may be stored in, for example, the storage unit 23.

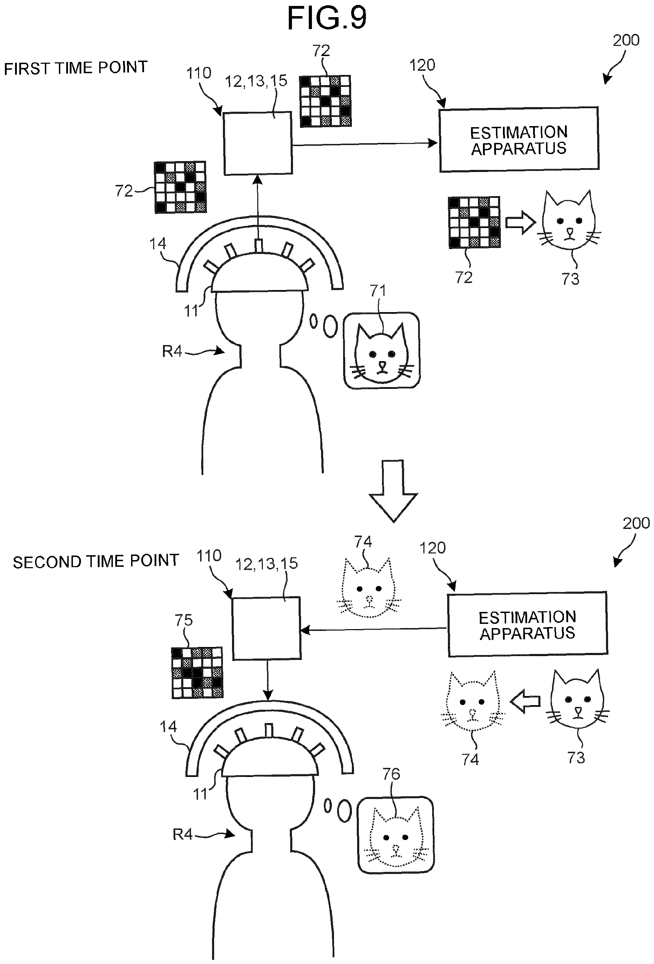

A sensory transmission method using the sensory transmission system 200 that is configured as described above will be described below. FIG. 9 is a diagram schematically illustrating an example of operation performed by the sensory transmission system 100. As illustrated in an upper part in FIG. 9, the detection unit 11 of the detection stimulus apparatus 110 detects cerebral activation information 72 on the target subject R4 who perceives a cat face and evokes sensory information 71. The communication unit 12 transmits the cerebral activation information 72 that is detected by the detection unit 11 to the estimation apparatus 120. In the estimation apparatus 120, the communication unit 21 receives the cerebral activation information 72 that is transmitted from the detection stimulus apparatus 110. The processing unit 22 inputs the received cerebral activation information to the seventh trained model or the eighth trained model. The seventh trained model or the eighth trained model outputs reference sensory information 73 that corresponds to the input cerebral activation information 72. The processing unit 22 acquires the output reference sensory information 73 as an estimation result. The storage unit 25 stores therein the acquired reference sensory information 73.

As illustrated in a lower part in FIG. 9, at the second time point later than the first time point, for example, when the target subject R4 attempts to relieve the experience of perception of the cat face as described above, the subject R4 performs preparation so as to be able to receive a stimulus from the stimulus application unit 14 of the detection stimulus apparatus 110. The processing unit 22 estimates corresponding sensory information 74 corresponding to the reference sensory information 73 on the target subject R4 based on the reference sensory information 73 that is stored in the storage unit 25. In this case, the processing unit 22 inputs the acquired reference sensory information 73 to the ninth trained model. The ninth trained model outputs the corresponding sensory information 74 that corresponds to the input reference sensory information 73. The processing unit 22 acquires the output corresponding sensory information 74 as an estimation result. The communication unit 21 transmits the acquired corresponding sensory information 74 to the detection stimulus apparatus 110.

In the detection stimulus apparatus 110, the communication unit 12 receives the corresponding sensory information 74 that is transmitted from the estimation apparatus 120. The processing unit 13 inputs the received corresponding sensory information 74 to the third trained model that is stored in the storage unit 15. The third trained model outputs stimulus image information 75 that corresponds to the input corresponding sensory information 74. The stimulus application unit 14 applies a stimulus to the brain of the target subject R4 by applying an electromagnetic wave to the target subject R4 based on the output stimulus image information 75. As a result, the target subject R4 to whom the stimulus is applied by the stimulus application unit 14 evokes corresponding sensory information 76 that corresponds to the stimulus image information 75. In other words, the target subject R4 evokes the visual information that was evoked by viewing the cat face at the first time point, as the corresponding sensory information 76 at the second time point. In this manner, the visual information on the cat face is transmitted from the target subject R4 at the first time point to the target subject R4 at the second time point. Consequently, the target subject R4 is able to relieve the experience of viewing the cat face.

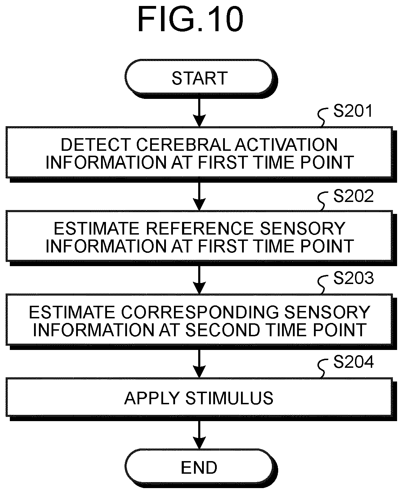

FIG. 10 is a flowchart illustrating operation performed by the sensory transmission system 200. As illustrated in FIG. 10, in the sensory transmission system 200, the detection stimulus apparatus 110 detects the cerebral activation information that the target subject R4 evoked at the first time point (Step S201). Subsequently, the estimation apparatus 20 estimates the reference sensory information at the first time point based on the cerebral activation information on the target subject R4 (Step S202). Then, the estimation apparatus 20 estimates the corresponding sensory information that corresponds to the reference sensory information on the second subject R2 that is different from the first subject R1 based on the estimated reference sensory information (Step S203). Subsequently, the second apparatus 30 applies a stimulus to the second subject R2 so as to evoke the estimated corresponding sensory information (Step S204).

As described above, the sensory transmission system 200 according to the present embodiment includes a detection apparatus (the detection stimulus apparatus 110) that detects cerebral activation information on the target subject R4 when the target subject R4 performs perception, the estimation apparatus 120 that estimates reference sensory information that is sensory information that is evoked in response to the perception based on the cerebral activation information on the target subject R4 that is detected at the first time point, and estimates corresponding sensory information that corresponds to the reference sensory information on the target subject R4 at the second time point later than the first time point based on the estimated reference sensory information, and the stimulus apparatus (the detection stimulus apparatus 110) that applies a stimulus to the target subject R4 at the second time point so as to evoke the estimated corresponding sensory information.

Furthermore, the sensory transmission method according to the present embodiment includes detecting, when the target subject R4 performs perception, cerebral activation information on the target subject R4, estimating reference sensory information that is sensory information that is evoked in response to the perception based on the cerebral activation information on the target subject R4 that is detected at the first time point and estimating corresponding sensory information that corresponds to the reference sensory information on the target subject R4 at the second time point later than the first time point based on the estimated reference sensory information, and applying a stimulus to the target subject R4 at the second time point so as to evoke the estimated corresponding sensory information.

With this configuration, the target subject R4 at the second time point is not caused to evoke the reference sensory information on the target subject R4 at the first time point as it is, but the corresponding sensory information that corresponds to the target subject R4 at the second time point is estimated based on the reference sensory information, and a stimulus is applied to the target subject R4 so as to evoke the estimated corresponding sensory information. Therefore, even when brain activities that evoke the sensory information vary between the target subject R4 at the first time point and the target subject R4 at the second time point, it is possible to appropriately transmit the sensory information.

In the sensory transmission system 200 according to the present embodiment, the reference sensory information is sensory information that is evoked by the target subject R4 at the first time point. With this configuration, the sensory information on the target subject R4 at the first time point and the sensory information on the target subject R4 at the second time point are directly associated with each other, so that it is possible to appropriately transmit the sensory information.

In the sensory transmission system 200 according to the present embodiment, the reference sensory information is the standard sensory information that is extracted based on pieces of sensory information that correspond to one another among a plurality of subjects. With this configuration, the standard sensory information that is extracted based on the pieces of sensory information that correspond to one another among the plurality of subjects is used as the reference sensory information, so that it is possible to effectively transmit the sensory information between the target subjects R4 at different time points.

In the sensory transmission system 200 according to the present embodiment, the standard sensory information is extracted based on pieces of sensory information that are evoked by a plurality of subjects who have brain growth conditions equivalent to the target subject R4. With this configuration, it is possible to effectively transmit the sensory information between the target subjects R4 at different time points based on the pieces of sensory information that are evoked by a plurality of subjects who have brain growth conditions equivalent to the target subject R4.

### Fifth Embodiment

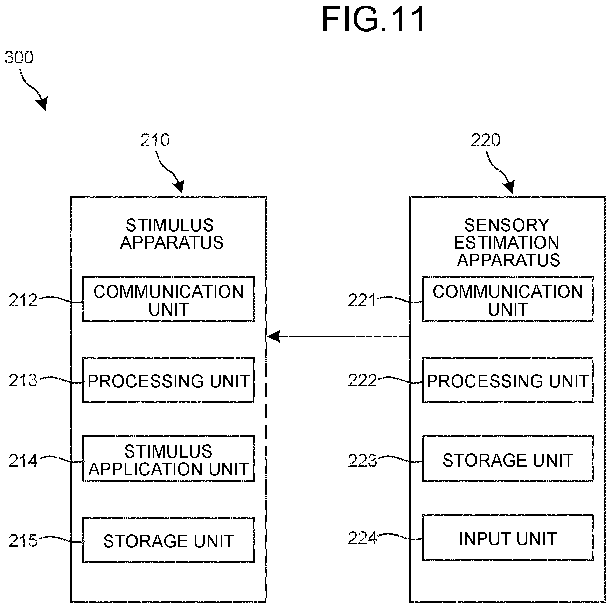

FIG. 11 is a diagram illustrating an example of a sensory transmission system 300 according to a fifth embodiment. As illustrated in FIG. 10, the sensory transmission system 300 according to the fifth embodiment includes a sensory estimation apparatus 220 and a stimulus apparatus 210.

The sensory estimation apparatus 220 includes a communication unit 221, a processing unit 222, a storage unit 223, and an input unit 224. The communication unit 221 performs wired communication or wireless communication with the stimulus apparatus 210. The storage unit 223 stores therein reference sensory information that is sensory information evoked in response to a perception. The storage unit 223 includes, for example, a storage, such as a hard disk drive or a solid state drive. Meanwhile, it may be possible to use an external storage medium, such as a removable disk, as the storage unit 223. The reference sensory information may be sensory information that a subject R5 or a person different from the subject R5 evokes in response to a perception, or standard sensory information that is extracted based on pieces of sensory information that correspond to one another among a plurality of subjects.

The processing unit 222 estimates corresponding sensory information that is sensory information corresponding to the reference sensory information on the subject R5, based on the reference sensory information that is stored in the storage unit 223. For example, it is possible to generate a tenth trained model by adopting, as a single training data set, sensory information on the subject R5 and sensory information corresponding to the reference sensory information, and performing machine learning on the training data set. The tenth trained model may be stored in, for example, the storage unit 223. When the input unit 224 (to be described later) designates the reference sensory information, the processing unit 222 estimates corresponding sensory information that corresponds to the designated reference sensory information, for example.

The input unit 224 is able to perform predetermined input operation for inputting information. As the input unit 224, for example, an input device, such as a keyboard or a touch pane, may be used. Meanwhile, it may be possible to use, as the input unit 224, a button, a lever, a dial, a switch, or a different input device in addition to or in place of the above-described input device. The input unit 224 is able to input sensory information that the subject R5 wants to experience from among a plurality of pieces of reference sensory information stored in the storage unit 223, for example.

The stimulus apparatus 210 includes a communication unit 212, a processing unit 213, a stimulus application unit 214, and a storage unit 215. The communication unit 212 is able to perform wired communication or wireless communication. The communication unit 212 receives the corresponding sensory information that is transmitted from the sensory estimation apparatus 220.

The processing unit 213 calculates stimulus image information corresponding to the received corresponding sensory information based on the corresponding sensory information that is received by the communication unit 212 and an eleventh trained model that has learned a correspondence relationship between the corresponding sensory information and the stimulus image information. The eleventh trained model may be the same trained model as the third trained model of the first embodiment as described above, for example. The stimulus application unit 214 applies a stimulus to the subject R5 by applying an electromagnetic wave signal to a target part in a brain of the subject R5 and activating the target part.

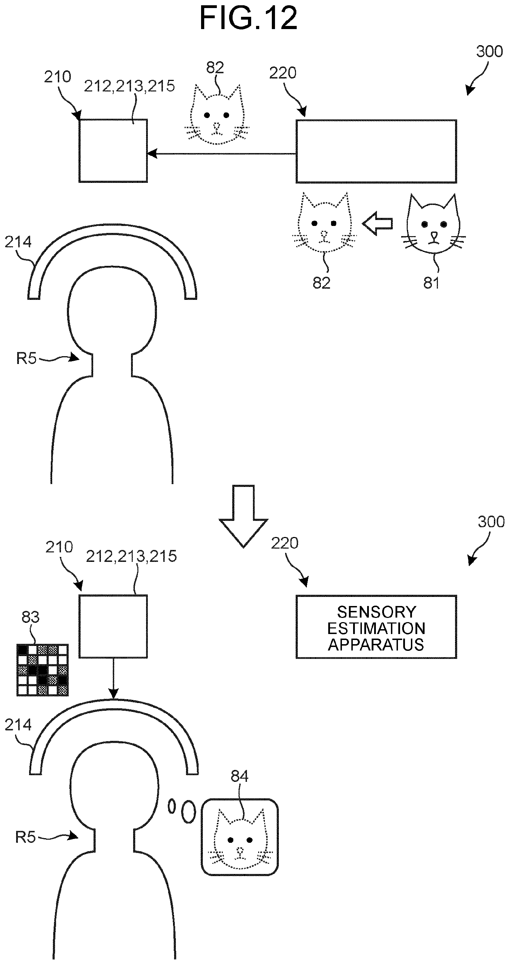

A sensory transmission method using the sensory transmission system 300 that is configured as described above will be described below. FIG. 12 is a diagram schematically illustrating an example of operation performed by the sensory transmission system 300 according to the present embodiment. The subject R5 performs preparation so as to be able to receive a stimulus from the stimulus application unit 214 of the stimulus apparatus 210. When the subject R selects reference sensory information 81 via the input unit 224, the processing unit 222 in the sensory estimation apparatus 220 estimates corresponding sensory information 82 on the subject R5 based on the selected reference sensory information 81. The processing unit 222 inputs, for example, the selected reference sensory information 81 to the tenth trained model. The tenth trained model outputs the corresponding sensory information 82 that corresponds to the input reference sensory information 81. The processing unit 222 acquires the output corresponding sensory information 82 as an estimation result. The communication unit 221 transmits the acquired corresponding sensory information 82 to the stimulus apparatus 210.

In the stimulus apparatus 210, the communication unit 212 receives the corresponding sensory information 82 that is transmitted from the sensory estimation apparatus 220. The processing unit 213 inputs the received corresponding sensory information 82 to the eleventh trained model that is stored in the storage unit 215. The eleventh trained model outputs stimulus image information 83 that corresponds to the input corresponding sensory information 82. The stimulus application unit 214 applies a stimulus to the subject R5 by applying an electromagnetic wave to the brain of the subject R5 based on the output stimulus image information 83. As a result, the subject R5 to whom the stimulus is applied by the stimulus application unit 214 evokes corresponding sensory information 84 that corresponds to the stimulus image information 83. In other words, the subject R5 evokes the visual information on the cat face as the corresponding sensory information 84. In this manner, the sensory estimation apparatus 220 transmits the visual information on the cat face to the subject R5.

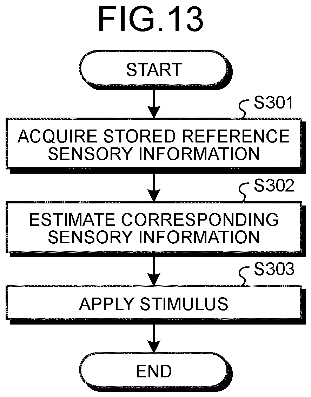

FIG. 13 is a flowchart illustrating operation performed by the sensory transmission system 300. As illustrated in FIG. 13, when the subject R5 selects the reference sensory information 81 via the input unit 224, the processing unit 222 in the sensory estimation apparatus 220 acquires the selected reference sensory information 81 (Step S301), and estimates the corresponding sensory information 82 on the subject R5 based on the acquired reference sensory information 81 (Step S302). Further, the stimulus apparatus 210 applies a stimulus to the fifth subject R5 so as to evoke the estimated corresponding sensory information 82 (Step S303).

As described above, the sensory estimation apparatus 220 according to the present embodiment includes the storage unit 223 that stores therein reference sensory information that is sensory information that is evoked in response to a perception, and the processing unit 222 that estimates corresponding sensory information that is sensory information corresponding to the reference sensory information on the subject R5 based on the reference sensory information that is stored in the storage unit 223.

Furthermore, a sensory estimation method according to the present embodiment includes acquiring reference sensory information from a storage unit 232 that stores therein reference sensory information that is sensory information that is evoked in response to a perception, and estimating corresponding sensory information that is sensory information corresponding to the reference sensory information on the subject R5 based on the acquired reference sensory information.

With this configuration, the corresponding sensory information that corresponds to the subject R5 is estimated based on the reference sensory information of the sensory estimation apparatus 220, so that it is possible to estimate appropriate sensory information for each of the subjects R5.

In the sensory estimation apparatus 220 according to the present embodiment, the reference sensory information is sensory information that a person different from the subject R5 evokes in response to a perception. With this configuration, it is possible to estimate appropriate sensory information between subjects who have different brain activities.

In the sensory estimation apparatus 220 according to the present embodiment, the reference sensory information is standard sensory information that is extracted based on pieces of sensory information that correspond to one another among the plurality of subjects R5. With this configuration, it is possible to appropriately transmit the sensory information among a large number of subjects.

The sensory transmission system 300 according to the present embodiment includes the sensory estimation apparatus 220 as described above, and the stimulus apparatus 210 that applies a stimulus to the subject R5 so as to evoke the corresponding sensory information that is estimated by the sensory estimation apparatus 220. With this configuration, it is possible to estimate appropriate sensory information for each of the subjects R5 and transmit the sensory information to the subject R5 based on the reference sensory information that is stored in the storage unit 223.

The technical scope of the present disclosure is not limited to the embodiments as described above, and appropriate modifications may be made within the scope not departing from the gist of the present disclosure. For example, in each of the embodiments as described above, the sensory information on the sense of sight has been explained as an example, but embodiments are not limited to this example, and different sensory information may be adopted as long as the sensory information enables detection of the cerebral activation information. Furthermore, for example, it may be possible to adopt specific sensory information that is obtained by cooperation of pieces of sensory information on five senses.

When a trained model is to be generated, it may be possible to adopt an instruction, such as "raise right hand" as a content to be provided to a subject, and it may be possible to learn reaction of a motor cortex of the subject. Further, when a trained model is to be generated, it may be possible to adopt a visual and auditory content, such as movie, as a content to be provided to the subject, and it may be possible to learn the entire brain of the subject.

Furthermore, it may be possible to assign an ID to an element of the standard sensory information when a trained model is to be generated, and assign a more similar ID with an increase in correlation with the content. For example, when contents of "dog", "cat", and "paper" are present, it may be possible to assign similar IDs to "dog" and "cat" and assign non-similar IDs to "dog" and "cat" and "paper".

Moreover, when the corresponding sensory information that corresponds to the standard sensory information is not present, it may be possible to adopt, as the corresponding sensory information, a content for which a similar ID is set as described above, or it may be possible not to transmit the corresponding sensory information.

Furthermore, it may be possible to replace the corresponding sensory information that is derived from certain standard sensory information with different sensory information and provide the different sensory information. For example, it may be possible to transmit, to a transmission destination, corresponding sensory information in which information is replaced with smell information on an orange, taste information on the orange, or tactile information on the orange, based on the standard sensory information corresponding to visual information on the orange.

Moreover, when the sensory information is to be transmitted from the first subject R1 to the second subject R2, it may be possible to delete command information indicating "transmission of sensory information to the second subject R2", and add communication control information corresponding to a packet header, such as "the first subject R1 evokes the sensory information" or "the first subject R1 requests the second subject R2 to evoke the sensory information".

Furthermore, when the standard sensory information is to be extracted, it is possible to perform online learning so as to continuously update a content. Moreover, it may be possible to store each piece of estimated sensory information in the storage unit and transmit each piece of the estimated sensory information to a transmission destination after a lapse of a predetermined period.

### Industrial Applicability

A sensory transmission system, a sensory transmission method, and a sensory transmission program according to the present disclosure may be used for a processing apparatus, such as a computer, for example.

### Reference Signs List

NW Neural network
R1 First subject
R2 Second subject
R4 Target subject
R5 Subject
S1 Convolutional layer
S2 Pooling layer
S3 Connected layer
10 First apparatus
11, 31 Detection unit,
12, 21, 32, 212, 221 Communication unit
13, 22, 33, 213, 222 Processing unit
14, 34, 214 Stimulus application unit
15, 23, 25, 35, 215, 223 Storage unit
20, 120 Estimation apparatus
30 Second apparatus
41, 47, 51, 61, 71 Sensory information
42, 52, 62, 72 Cerebral activation information
43, 53, 73, 81 Reference sensory information
44, 46, 54, 56, 64, 66, 74, 76, 82, 84 Corresponding sensory information
45, 55, 65, 75, 83 Stimulus image information
63 Standard sensory information
100, 200, 300 Sensory transmission system
110 Detection stimulus apparatus
130 Third apparatus
210 Stimulus apparatus
220 Sensory estimation apparatus
224 Input unit

## Claims

1. A sensory transmission system comprising:
a first apparatus configured to detect cerebral activation information on a first subject when the first subject performs perception;
an estimation apparatus configured to estimate reference sensory information that is sensory information that is evoked in response to the perception based on the detected cerebral activation information on the first subject and estimate corresponding sensory information that is sensory information corresponding to the reference sensory information on a second subject different from the first subject based on the estimated reference sensory information; and
a second apparatus configured to apply a stimulus to the second subject so as to evoke the estimated corresponding sensory information.

2. The sensory transmission system according to claim 1, wherein
the second apparatus is configured to detect cerebral activation information on the second subject when a stimulus is applied to the second subject,
the estimation apparatus is configured to estimate the reference sensory information based on the detected brain activation information on the second subject, and estimate the corresponding sensory information on the first subject based on the estimated reference sensory information; and
the first apparatus is configured to apply a stimulus to the first subject so as to evoke the estimated corresponding sensory information.

3. The sensory transmission system according to claim 1 or 2, wherein the reference sensory information is the sensory information that a subject for whom the cerebral activation information is detected between the first subject and the second subject evokes in response to the perception.

4. The sensory transmission system according to claim 1 or 2, wherein the reference sensory information is standard sensory information that is extracted based on the sensory information that correspond to one another among a plurality of subjects.

5. The sensory transmission system according to claim 1, wherein
the first apparatus includes a detection apparatus configured to detect cerebral activation information on a target subject when the target subject performs perception,
the estimation apparatus is configured to estimate reference sensory information that is sensory information that is evoked in response to the perception based on the cerebral activation information on the target subject that is detected at a first time point, and estimate corresponding sensory information that corresponds to the reference sensory information on the target subject at a second time point later than the first time point based on the estimated reference sensory information, and
the second apparatus includes a stimulus apparatus configured to apply a stimulus to the target subject at the second time point so as to evoke the estimated corresponding sensory information.

6. The sensory transmission system according to claim 5, wherein the reference sensory information is the sensory information that the target subject evokes in response to the perception at the first time point.

7. The sensory transmission system according to claim 5, wherein the reference sensory information is standard sensory information that is extracted based on the sensory information that correspond to one another among a plurality of subjects.

8. The sensory transmission system according to claim 7, wherein the standard sensory information is extracted based on the sensory information that are evoked by a plurality of subjects who have brain growth conditions equivalent to a brain growth condition of the target subject.

9. The sensory transmission system according to claim 1, wherein the estimation apparatus includes
a storage unit configured to store therein the reference sensory information, and
a processing unit configured to estimate the corresponding sensory information based on the reference sensory information that is stored in the storage unit.

10. The sensory transmission system according to claim 9, wherein the reference sensory information is the sensory information that a person different from the first subject evokes in response to the perception.

11. The sensory transmission system according to claim 9, wherein the reference sensory information is standard sensory information that is extracted based on the sensory information that correspond to one another among a plurality of subjects.

12. A sensory transmission method comprising:
detecting cerebral activation information on a first subject when the first subject performs perception;
estimating reference sensory information that is sensory information that is evoked in response to the perception based on the detected cerebral activation information on the first subject;
estimating corresponding sensory information that is sensory information corresponding to the reference sensory information on a second subject different from the first subject based on the estimated reference sensory information; and
applying a stimulus to the second subject so as to evoke the estimated corresponding sensory information.

13. A sensory transmission program that causes a computer to perform processing including:
detecting cerebral activation information on a first subject when the first subject performs perception;
estimating reference sensory information that is sensory information that is evoked in response to the perception based on the detected cerebral activation information on the first subject;
estimating corresponding sensory information that is sensory information corresponding to the reference sensory information on a second subject different from the first subject based on the estimated reference sensory information; and
applying a stimulus to the second subject so as to evoke the estimated corresponding sensory information.
